# EUROPEAN PATENT SPECIFICATION

(11) **EP 4 377 287 B1**
(45) Date of publication and mention of the grant of the patent: **30.04.2025**
(21) Application number: 22743517.9
(22) Date of filing: 28.07.2022
(51) Int. Cl.: C07C 51/44, C07C 51/487, C07C 51/02, C07C 51/47, C07C 59/08, C07C 51/41, B01D 61/44, C12P 7/56, C25B 1/16, B01D 61/42

(54) **PROCESS FOR MANUFACTURING LACTIC ACID**
VERFAHREN ZUR HERSTELLUNG VON MILCHSÄURE
PROCÉDÉ DE FABRICATION D'ACIDE LACTIQUE

(30) Priority: 30.07.2021 EP 21188937
(43) Date of publication of application: 05.06.2024
(73) Proprietor: Purac Biochem B.V., 4206 AC Gorinchem (NL)
(72) Inventor: VAN STRIEN, Cornelis, Johannes, Govardus, 4206 AC Gorinchem (NL); MERLET, Renaud, Benoit, 4206 AC Gorinchem (NL)
(74) Representative: De Vries & Metman
(86) International application number: PCT/EP2022/071195
(87) International publication number: WO 2023/006876

(56) References cited:
- WO-A1-2005/123647
- WO-A1-2011/095631

## Description

An aspect of the present invention pertains to a process for manufacturing lactic acid in high purity in an economical manner.

Lactic acid is often manufactured via fermentation of carbohydrates by micro-organisms. A common feature to all fermentation processes is the need to neutralise the acids excreted by the micro-organisms. A drop in pH below a critical value, depending on the micro-organism used in the process, could damage the micro-organism's metabolic process and bring the fermentation process to a stop. Therefore, it is common practice to add a base in the fermentation media in order to control the pH. This results in the lactic acid produced being present in the fermentation media in the form of a lactate salt.

Despite the longstanding practice to produce lactic acid via fermentation, one of the challenges in the manufacture of lactic acid is still to obtain the acid in a relatively pure form while at the same time carrying out the process in an economical manner on a scale which is commercially attractive.

Electrodialysis is one of the purification processes that may be used in the production of lactic acid via fermentation. Water-splitting electrodialysis in particular allows the direct conversion of the lactate salt into lactic acid and base. In this type of electrodialysis bipolar membranes are generally used to split water into H⁺ and OH⁻ respectively, which combine with the anion and cation of the lactate salt respectively, resulting in the production of separate solutions of lactic acid and base.

The use of water-splitting electrodialysis on aqueous media provided by fermentation, in particular for manufacturing organic acids, has been limited by the necessity to remove fermentation-derived products from the feed (e.g. sugar, protein and amino acids). Such fermentation derived matter negatively interferes with the water-splitting electrodialysis process by, for instance, fouling of the ion-permeable membranes and decreasing power efficiency.

WO2011095631 is directed to a process for the preparation of lactic acid comprising the steps of:
a) providing an aqueous medium comprising magnesium lactate;
b) adding to the aqueous medium comprising magnesium lactate a monovalent base to form an aqueous medium comprising a water soluble monovalent lactate salt and a solid magnesium base;
c) separating the magnesium base from the aqueous medium comprising the water soluble monovalent lactate salt;
d) adjusting the concentration of the monovalent lactate salt in the aqueous medium to a value between 10 and 30 wt.%,
e) subjecting the aqueous medium comprising the monovalent lactate salt to water-splitting electrodialysis, to produce a first solution comprising monovalent base and a second solution comprising lactic acid and monovalent lactate salt, the electrodialysis being carried out to a partial conversion of 40 to 98 mole%;
f) separating the second solution comprising lactic acid and monovalent lactate salt into lactic acid and a solution comprising the monovalent lactate salt by vapour-liquid separation;
g) recycling the solution of step f) comprising the monovalent lactate salt to step d).

There is still need for a process for manufacturing lactic acid which provides lactic acid in high purity in high yield, which can be performed in an economical manner with low power consumption while minimising the production of substantial amounts of non-reusable components (i.e. waste by-products).

An aspect of the present invention pertains to a process for the preparation of lactic acid comprising the steps of:
a) providing an aqueous medium comprising magnesium lactate;
b) adding to the aqueous medium comprising magnesium lactate a monovalent base to form an aqueous medium comprising a water soluble monovalent lactate salt and a solid magnesium base;
c) separating the solid magnesium base from the aqueous medium comprising the water soluble monovalent lactate salt;
d) providing an aqueous medium comprising the water soluble monovalent lactate salt at a concentration of more than 30 and at most 45 wt.%;
e) subjecting the aqueous medium comprising the water soluble monovalent lactate salt from step d) to water-splitting electrodialysis, to produce a first solution comprising monovalent base and a second solution comprising lactic acid and monovalent lactate salt, the electrodialysis being carried out to a partial conversion of 40 to 99 mole%;
f) recovering lactic acid from the second solution comprising lactic acid and monovalent lactate salt.

Carrying out the water-splitting electrodialysis to a partial conversion of 40 to 99 mole%, in particular of 40 to 98 mole% and, if so desired, subsequently recycling the remaining lactate salt to the electrodialysis step advantageously results in an optimal process with low power consumption in combination with high yield.

Furthermore, the process as described herein produces virtually no waste by-products, since all compounds formed and separated in the different steps may be recycled. The magnesium base separated in step c) may for instance be used in the fermentation process and the solution comprising monovalent base of step e) may be used in the salt exchange step b). Recovery step f) generally generates a solution comprising monovalent lactate salt and optionally lactic acid. This solution can, if so desired, be recycled at least in part to any of the process steps preceding the water-splitting electrodialysis, e.g., to one or more of step b), c), d), and e).

In step d) an aqueous medium is provided comprising the water soluble monovalent lactate salt at a concentration of more than 30 to at most 45 wt.%. This step may comprise a concentration step, if the product of step c) has a concentration step which is unattrac-tively low. This step may also comprise a dilution step if very high concentrations are obtained in step c). If the product from step c) already has a concentration in the desired range, no further concentration adjustment is required. In that case step d) will generally comprise nothing more than providing the product of step c) to step e).

It is preferred for the concentration of the monovalent lactate salt concentration in the aqueous medium to be relatively high, as this will ensure that less water has to be removed from the system in order to isolate the lactic acid. Additionally, it has been found that the use of relatively high monovalent lactate salt concentrations improves the performance of the water-splitting electrodialysis step, in particular when the monovalent lactate salt is potassium lactate.

Accordingly, it is preferred for the aqueous medium comprising the water soluble monovalent lactate salt from step d) to have a water soluble monovalent lactate salt concentration of at least 32 wt.%. If the concentration is too high, it may become difficult to perform water-splitting electrodialysis. In some embodiments the current efficiency will be affected. It may therefore be preferred for the monovalent lactate salt concentration to be at most 40 wt.%. Further preferences will be discussed below.

The preferences expressed above apply to all monovalent lactate salts, but is particularly attractive if the monovalent lactate salt is potassium lactate, as will be discussed in more detail below.

The aqueous medium comprising a magnesium lactate salt may preferably be provided by a fermentation process. The magnesium lactate salt is generally already present in an aqueous medium when it leaves the fermentation. In such a process, a carbohydrate source is fermented to lactic acid by means of a lactic acid-producing micro-organism. During fermentation, a magnesium base is added as neutralising agent. This results in the formation of an aqueous medium comprising the corresponding magnesium lactate salt.

The base anion of the magnesium base is preferably chosen from at least one of hydroxide, carbonate and hydrogencarbonate, and more preferably is hydroxide. Although the use of magnesium as the base cation is preferred, another alkaline earth metal cation, such as a calcium cation, may also be used. The amount of alkaline earth metal base added is determined by the amount of lactic acid produced and may be determined via pH control of the fermentation medium.

The biomass (i.e. microbial cell matter) may be removed from the fermentation broth before further processing of the lactate-containing medium. Biomass removal may be effected, for example, by conventional methods including filtration, flotation, sedimentation, centrifugation, flocculation and combinations thereof. Separation of biomass from solid magnesium lactate crystals may also be carried out using a hydrocyclone. It is within the scope of the skilled person to determine an appropriate method. Other optional treatments prior to further processing include washing, filtration, (re)crystallisation, concentration and combinations thereof.

Magnesium is a preferred alkaline earth metal, since the use of a magnesium base advantageously results in the formation of magnesium lactate in an appropriate crystalline form to enable separation of the crystalline material from the fermentation broth including the biomass. Separation of the magnesium lactate may be done by any known processing technique for solid/liquid separations. It may be done for instance via filtration using a filter with a suitable pore size to retain magnesium lactate on the filter and to enable subsequent removal of impurities by washing of the filter cake. The above-mentioned biomass separation is in principle not needed unless there is the wish to further process or (re-)use the remaining fermentation broth for specific purposes.

The thus purified magnesium lactate is especially suitable for further processing as described herein and in particular when using water-splitting electrodialysis, wherein fermentation-derived products (e.g. sugar, protein, amino acids) may negatively interfere by, for instance, increasing the power consumption and fouling of the ion-permeable membranes.

The aqueous medium comprising the alkaline earth metal lactate salt, preferably the magnesium lactate salt, is subjected to a salt exchange reaction (step b), wherein a monovalent base is added to said aqueous medium to form a monovalent lactate salt and a solid alkaline earth metal base.

See also WO 2005/123647, describing the use of a magnesium base in lactic acid fermentation and the salt exchange reaction between magnesium lactate and a monovalent base.

If the aqueous medium containing the alkaline earth metal lactate is provided by fermentation, the base anion is generally chosen to correspond to the base anion used as neutralising agent during fermentation.

The monovalent base added is preferably one or more of a hydroxide, carbonate or hydrogencarbonate, more preferably a hydroxide, of a monovalent cation, the monovalent cation being sodium, potassium, lithium, ammonium, monoalkylammonium, dialkylammonium, trialkylammonium, or tetraalkylammonium, preferably sodium or potassium and more preferably sodium. The use of sodium and potassium bases advantageously results in a higher conversion of the alkaline metal earth lactate salt to the monovalent lactate salt than when ammonium bases are used. This is relevant for preparing a product with a low alkaline earth metal ion content suitable for water-splitting electrodialysis. The residual alkaline earth metal ions may nonetheless be removed by methods known to the skilled person, such as the use of ion exchange resins.

The use of potassium may be particularly preferred with regard to advantages associated to a subsequent water-splitting electrodialysis of the resulting potassium lactate salt, as explained in more detail below, when combined with a concentration of more than 30 wt.% potassium lactate, even more in particular more than 32 wt.%, in particular more than 33 wt.%.

The amount of monovalent base is determined by stoichiometric and pH considerations. It may be preferred to use a surplus of base to obtain a high conversion and to ensure the removal of virtually all alkaline earth metal ions from the lactate. In general, it is preferred to perform the salt exchange reaction in two steps, wherein in the first step the pH is between 9 and 12, preferably between 9.5 and 11, and in the second step the pH is slightly increased to a pH between 10.5 and 12.

The alkaline earth metal base formed in the salt exchange reaction typically is in solid form while the monovalent lactate salt is dissolved in the aqueous medium. The two components may therefore be separated by conventional solid-liquid separation processes, such as one or more of filtration, sedimentation, and centrifugation.

If so desired, the alkaline earth metal base obtained after separation may be recycled to the fermentation process, optionally after washing to remove residual alkaline salt.

Additional treatments, such as one or more of ion exchange treatment, activated carbon treatment, desalting electrodialysis, dilution, concentration (e.g. thermal or using a membrane) and filtration (e.g. nanofiltration) may be performed prior to water-splitting electrodialysis. For instance, as a safety measure to prevent a too high alkaline earth metal level in the aqueous medium comprising the monovalent lactate salt, an ion exchange step may be performed prior to electrodialysis to lower the alkaline earth metal content thereof.

However, in some embodiments the process as described herein advantageously does not necessitate such additional treatments, especially when the lactate is provided via fermentation and a magnesium base is added in the fermentation process to provide magnesium lactate fermentation broth. In particular, the use of a magnesium base for neutralization during fermentation, which as discussed above provides magnesium lactate in an appropriate crystalline form, has been found to preclude the need of further purification steps generally required to remove the fermentation derived matter (e.g. sugar, protein and amino acid) from feeds for use in water-splitting electrodialysis. This advantageously lowers the complexity, power demand and costs generally associated to such electrodialytical processes.

The aqueous medium comprising the monovalent lactate salt is then subjected to water-splitting electrodialysis.

As indicated above, the aqueous medium comprising the water soluble monovalent lactate salt from step d) has a water soluble monovalent lactate salt concentration of more than 30 wt.% and at most 45 wt.%, in particular more than 32 wt.%. This is the initial concentration in the aqueous medium that is subjected to electrodialysis (the feed solution). It may be preferred for the monovalent lactate salt concentration to be at most 40 wt.%. In one aspect, the initial concentration is preferably from 32 to 38 wt%, even more preferably from 34 to 36 wt.%, as a most preferred concentration an initial concentration of about 35 wt.% may be mentioned.

Depending on the salt concentration, the aqueous medium comprising the monovalent lactate salt as obtained after the salt exchange reaction, i.e. after the separation step c), may be used directly as feed to the electrodialysis, or, if necessary, may be diluted or concentrated to adjust the salt concentration prior to water-splitting electrodialysis. Concentration may be carried out by for instance evaporation or conventional electrodialysis. Concentration may be preferably performed by evaporation (e.g. evaporation of water). Concentration by evaporation may be particularly preferred when concentrating to monovalent lactate salt concentrations of more than 30 wt.%. Evaporation may be preferably performed in an evaporator with mechanical vapour recompression. Alternatively, membrane concentration may also be used, or a combination of evaporation and membrane concentration.

Starting with a higher monovalent lactate salt concentration (e.g. of more than least 30 wt.%) the lactic acid concentration of the resulting solution obtained after water-splitting electrodialysis will also be higher.

For instance, the lactic acid concentration in the second solution obtained by water-splitting electrodialysis may be from 14 wt.% to 35 wt.%. Depending on the operating conditions, the lactic acid concentration preferably is at least 20 wt.%, in particular at least 22 wt.%, more in particular at least 25 wt.%. A preferred upper limit may be 30 wt.%. In particular embodiments, the upper limit may be at most 29 wt.%, or 28 wt.%, or even 27 wt.%. A range of 25-30 wt.% is considered generally preferred.

This may be particularly advantageous when aiming to isolate lactic acid. In such a situation, all water must be removed from the system. It has now been found that starting with concentrations from at least 30 wt.%, e.g., in the range of 30 to 40 wt.% of monovalent lactate salt, advantageously allows for the removal of water prior to water-splitting electrodialysis, e.g. during a concentration-adjusting step to increase monovalent lactate salt concentration. This is achieved without detrimentally affecting the performance of the water-splitting electrodialysis or, as explained in more detail below, whilst even improving the water-splitting electrodialysis process.

Removal of water prior to the water-splitting electrodialysis step means that less water needs to be removed after the water-splitting electrodialysis. This advantageously results in an improved method since the removal of water from a lactic acid containing solution (such as the solution obtained after water-splitting electrodialysis) is more costly and cumbersome than the removal of water from a monovalent lactate salt (such as the starting solution prior to water-splitting electrodialysis). In particular, water removal from a lactic acid solution will have more corrosion issues associated thereto and requires more costly equipment.

Accordingly, in several aspects of the invention, prior to performing the water-splitting electrodialysis in a method as described herein, adjusting the concentration of the monovalent lactate salt in the aqueous medium to a value of more than 30 wt.% and at most 45 wt.% comprises a water removal step. Water removal may be preferably performed in an evaporator with mechanical vapour recompression.

It has also been found that an initial concentration of the monovalent lactate salt within the above indicated range of more than 30 wt.% and preferably below 40 wt.% results in an improved performance of the water-splitting electrodialysis step. This may be particularly so when the monovalent lactate salt is potassium lactate.

It has been found that starting with a monovalent lactate salt with the above indicated concentration the water-splitting electrodialysis may be performed to a given degree of conversion (e.g. up to 99 mole% or up to 98 mole %) while using less energy than required for the same degree of conversion when starting with monovalent lactate salt solutions of lower concentration.

Similarly, starting with a monovalent lactate salt with the above indicated concentration the water-splitting electrodialysis may be performed to a higher degree of conversion for the same amount of energy as required to achieve a lower degree of conversion when starting with lower monovalent lactate salt concentrations.

Starting solutions with monovalent lactate salt concentrations of higher than 40 wt.% may detrimentally affect the current efficiency of the water-splitting electrodialysis.

The concentration of the monovalent lactate salt in the aqueous medium may be determined by methods known to the skilled person, for instance by using conductivity measurements or Inductively Coupled Plasma mass spectrometry analysis, or methods like titration or Brix (refractometry).

The water-splitting electrodialysis is carried out to a partial conversion of 40 to 99 mole%. It may be preferred for the partial conversion to be at least 50 mole%, in particular at least 60 mole%, more in particular at least 70 mole%, still more in particular at least 80 mole%, especially at least 85 mole%, or at least 90 mole%. It may be preferred for the partial conversion to be at most 98 mole%. Therefore, in one embodiment water-splitting electrodialysis is carried out to a partial conversion of 90-98 mol.%, in particular 90-95 mole%. In other embodiments the water-splitting electrodialysis may be performed to a conversion from 98 to 99 mole %, in particular higher than 98 mole% and of at most 99 mole%.

A first solution comprising monovalent base and a second solution comprising lactic acid and monovalent lactate salt are produced in this process.

A partial conversion of 40 to 99 mole% means that 40 to 99 mole% of the monovalent lactate salt present in the feed solution is converted into lactic acid. This results in the second solution produced by the electrodialysis comprising lactic acid in an amount of 40 to 99 mole%, calculated on the total molar amount of lactic acid and lactate present in the solution.

The degree of conversion may be monitored by measuring conductivity of the second solution using methods known to the person skilled in the art.

In addition to the conversion level and the initial salt concentration of the feed solution, the conductivity of the second solution will depend on the temperature of the electrodialysis process. The higher the temperature at which the electrodialysis is performed, the lower the power consumption will be. Hence, the working temperature is chosen to optimise power consumption without compromising the performance and the life of the ion-specific permeable membranes. In general, values between 25 and 80°C may be mentioned. Conventionally, the water-splitting electrodialysis is performed at a temperature between 25 °C and 40 °C. However, it is possible to conduct the electrodialysis at higher temperatures, e.g. above 40°C, such as at least 45°C, or at least 50 °C, for instance between 60 °C and 80 °C, to allow for a low power consumption and the possibility for heat recovery.

The water-splitting electrodialysis as described herein may be performed using a conventional apparatus and conventional methods. Preferably the water-splitting electrodialysis is carried out in an electrodialysis apparatus provided with a cation exchange membrane and a bipolar membrane.

In one embodiment, a water-splitting electrodialysis cell comprises a two compartment unit. The aqueous medium comprising the monovalent lactate salt is introduced in the salt/acid compartment (or feed compartment). The monovalent cations are transported from the salt/acid compartment to the base compartment through the cation exchange membrane to produce the first solution comprising the monovalent base. Simultaneously, H⁺ ions are transported to the salt/acid compartment to produce the second solution comprising lactic acid and monovalent lactate salt.

In another embodiment, a water splitting electrodialysis cell comprises a three compartment unit, comprising a salt compartment, a base compartment, and an acid compartment. The aqueous medium comprising the monovalent lactate salt is introduced in the salt compartment (or feed compartment). The monovalent cations are transported from the salt compartment to the base compartment through the cation exchange membrane to produce the first solution comprising the monovalent base. Simultaneously, lactate anions are transported from the salt compartment to the acid compartment through the anion exchange membrane to produce the second solution comprising the lactic acid. As the monovalent salt ions are transported into their respective compartments, H+ and OH- ions are transported from the bipolar membrane to the acid and base compartments, respectively.

It is preferred to apply the water-splitting electrodialysis to monovalent lactate salts of sodium and potassium. When using ammonium lactate, care must be taken to control the emission of toxic ammonia resulting from the generation of ammonium hydroxide. Monovalent lactate salts of potassium may be particularly preferred as potassium lactate has been found to further improve the water-splitting electrodialysis step, by requiring lower energy consumption than other lactate salts, e.g., sodium lactate. This may be to the finding that potassium lactate solutions have a higher conductivity than sodium lactate solutions. The maximum conductivity of potassium lactate solutions has been identified at a concentration of more than 30 wt.% to about 45 wt.%, in particular 40 wt.%, in particular at about 35 wt.%. Without being bound to any theory, this higher conductivity may explain lower voltage drops and lower energy use when working with potassium lactate. Potassium lactate solutions have also been found to have a lower viscosity than, e.g., sodium lactate solutions, which advantageously results in lowering pressure drops and in lowering the requirements on pumping energy when using the equipment in a water-splitting electrodialysis as described herein.

A method as described herein further comprises recovering lactic acid from the second solution, produced by the water-splitting electrodialysis, comprising lactic acid and monovalent lactate salt.

In several embodiments, the second solution produced by the water-splitting electrodialysis is separated into lactic acid and a solution comprising the monovalent lactate salt. The separation may be achieved by one or more of vapour-liquid separation, liquid-liquid separation and solid-liquid separation. Ion exchange methods, e.g. the use of ion exchange resins, may also be used to recover lactic acid from the second solution produced by the water-splitting electrodialysis.

In one embodiment the lactic acid is separated from the monovalent lactate salt by means of vapour-liquid separation.

Vapour-liquid separation may be performed by distillation or evaporation. Distillation is preferred, since it provides a substantially complete separation of the acid from the salt. The solution comprising lactic acid and monovalent lactate salt may be concentrated prior to distillation. Distillation is preferably carried out in a vacuum distillation unit. Vacuum distillation is found to be especially suited to separate lactic acid from lactate salt in a situation where the mixture of lactic acid and lactate salt is obtained by water-splitting electrodialysis being carried out to a partial conversion as described herein and where magnesium base is used for neutralisation in fermentation.

A suitable process and/or apparatus for concentration and vacuum distillation is described in WO 01/38283. The distillation process may comprise two or more distillation steps, the first distillation step preferably being carried out at a temperature from 80 to 150 °C, preferably from 100 to 140 °C, and a pressure between 40 and 250 mbar, preferably between 50 and 150 mbar, and the second distillation step preferably being carried out at a temperature from 80 to 200 °C, preferably from 100 to 200 °C, and a pressure between 0.01 and 50 mbar, preferably between 0.1 and 20 mbar.

The first distillation step may comprise a combination of one or more film evaporators with distillation columns and serves the purpose of concentrating the lactic acid/lactate salt product stream as high as possible.

The second distillation step may also comprise a combination of one or more film evaporators with one or more distillation units. In the second distillation step, the majority of the lactic acid in the product of the first distillation step is distilled, preferably under vacuum, forming a top fraction comprising the majority of the lactic acid and a distillation residue (bottom fraction) comprising the monovalent lactate salt. The second distillation step may be carried out in one or more short path distillation (SPD) devices having an inner condenser. However, in order to reduce contamination by splashing of impurities into the condensed lactic acid, i.e. to minimise the alkali metal content in the distilled lactic acid product, the use of a vacuum distillation unit as described in Figures 5A and 5B of the above-mentioned WO 01/38283 (see page 10, line 17, to page 11, line 7) is preferred, as this specific set up prevents any splashing from taking place. Preferably, the second distillation step comprises a film evaporator (preferably a falling film, a wiped film or a thin film evaporator) that is at the bottom of the evaporator directly in connection with- or is connected via a specifically U-shaped connection with- a vacuum distillation unit comprising a packing and preferably a cooling device so that it may be operated under reflux. In the film evaporator, the lactic acid is brought in vapour phase after which it enters the vacuum distillation through the connection at the bottom where it subsequently is distilled.

It is further preferred for the product from the first distillation step (the first bottom fraction) to be subjected to a conditioning step (so-called "preflash") before it undergoes a second distillation step, the pressure in this conditioning step preferably being the same as that used in the second distillation. This preferred embodiment has the advantage that a residual quantity of water and dissolved gases are removed before the product is subjected to the second distillation step. The use of a preflash allows the lactic acid/monovalent lactate content to be increased so that in the second distillation step it is possible to obtain both a purer lactic acid product and to achieve more stable operation.

Liquid-liquid separation may comprise extraction, and, for the recovery of lactic acid from the loaded solvent, back extraction, or other techniques. Liquid-liquid separation may also comprise filtration, e.g., ultrafiltration, microfiltration, nanofiltration, reverse osmosis, decantation, diffusion dialysis or donnan dialysis.

Solid-liquid separation may comprise a crystallisation step. For example, the lactic acid may be crystallised in a static crystallisation unit, by fractional crystallisation, by suspension crystallisation and/or by wash column crystallisation. The crystals may then be separated from the liquid phase of the solution crystals by filtration or centrifugation. The crystallisation may comprise one or more of a concentration step, such as a water evaporation step, a cooling step, a seeding step, and one or more washing steps. Solid-liquid separation, and in particular crystallisation, has the disadvantage that, in order to ensure a product of high purity, the yields of recovery are generally low. For instance, in some processes after a first crystallisation the yield of recovery of lactic acid is about 46% and the purification factor is 15-20, which is the ratio of the amount of impurities in the product before and after crystallisation. To achieve a purification factor of between 100-160, a second crystallisation step is required and the overall yield then drops to 22 %.

Lactic acid may also be recovered from the second solution comprising lactic acid and monovalent lactate salt, by an ion exchange treatment, whereby residual monovalent cations in the second solution are replaced by protons by using, e.g., ion exchange resins. This method may be particularly suited for instance, when the water-splitting electrodialysis is performed to a high degree of conversion, of for instance at least 90 mole%, in particular at least 95 mole%, e.g., 98 to 99 mole %.

The solution containing the monovalent lactate salt obtained after separation can be processed as desired. It can, if so desired, be recycled to earlier steps in the process so that it is provided to water-splitting electrodialysis. It can, e.g., be recycled at least in part to one or more of steps b), c), d), and e). This recycling step ensures that no substantial yield loss is suffered as a consequence of the partial conversion of the lactate into lactic acid during water-splitting electrodialysis.

The lactic acid product obtained after recovery step f) may be in solid form, liquid form or in solution, and generally comprises at least 95 wt.% of lactic acid, preferably at least 97 wt.% of lactic acid, more preferably at least 99 wt.% of lactic acid, even more preferably at least 99.5 wt.% of lactic acid and most preferably at least 99.9 wt.% of lactic acid. The lactic acid obtained by the process according to an aspect of the invention is therefore of high purity and is suitable for direct use in, for example, synthetic processes, food applications and cosmetic applications.

The lactic acid obtained is especially suited for the preparation of lactide and/or polylactic acid, wherein during the polymerisation of lactic acid the presence of impurities, such as lactate salts, may result in undesirable racemisation of lactic acid moieties leading to a lactide and polylactic acid product of lower quality. In general the amount of metal ions should be below 5 ppm. For instance, the presence of sodium sulfide, in quantities as low as 20 ppm, in lactic acid negatively affects the optical purity of the polylactic acid product.

Any conventional process as known to the person skilled in the art may be used for said manufacture of lactide and/or polylactic acid provided that the starting material containing lactic acid is made via the process as described herein.

The process as described herein advantageously is accompanied by a low power consumption and ensures that no or substantially no waste by-products are generated.

Aspects of the present invention are further illustrated by the following Examples, without being limited thereto or thereby.

### Example 1: Partial electrodialysis of sodium lactate solution.

An Electrocell electrodialysis module (Sweden) was equipped with a Fu-matech FBM bipolar membrane, and a Neosepta CMB cation exchange membrane. A set-up with two electrode compartment and one feed compartment was used. The membrane areas of the bipolar and the cation exchange membrane was 0.01 m². The first compartment comprised of the anode and the cation exchange side of the bipolar membrane, the second feed compartment of the anion exchange side of the bipolar membrane and the cation exchange membrane, and the third compartment of the cation exchange membrane and the cathode. 2 wt.% sulphuric acid in water was circulated through the anode compartment to ensure a high conductivity. A 20 wt.% sodium lactate solution was circulated through the middle compartment as a feed. A 8 wt.% sodium hydroxide solution was circulated through the cathode compartment to ensure a high conductivity at the cathode side, and to collect the sodium hydroxide produced. The three solutions were circulated with a peristaltic pump at 250 ml/min from a 500 ml glass buffer over the electrodialysis module. The glass buffer vessel were double walled and the temperature across the three compartments was kept between 40 and 60 °C with a water bath. The sulphuric acid, sodium hydroxide were reagent grade, and the Purac sodium lactate was of high purity food grade quality.

The electrodialysis experiment was carried out a constant 7.5 A DC current. In the experiment the sodium lactate solution in the feed compartment of the module was acidified batch wise through sodium removal through the cation exchange membrane to form sodium hydroxide in the cathode compartment, while protons generated by the bipolar membrane formed lactic acid with the original lactate ions.

The experiment lasted for about 230 min, when all lactate was converted. In the beginning of the experiment the sodium lactate solution had a high conductivity and the voltage was relatively constant at 9.5 to 10.5 V. After 180 min 80% of the sodium lactate was converted to lactic acid, with a residual sodium content of 0.84 wt.%, and the voltage increased to 12 V. The pH of the solution decreased to 3.1 from an initial pH of 6.0. After 210 min the conversion had increased to 94%, the residual sodium content had decreased to 0.25 wt.%, the voltage had increased to 16 V, and the pH had decreased to 2.56. After 225 min the conversion had increased to 98%, the residual sodium content had decreased to 0.06 wt.%, the voltage had increased to 22.4 V, and the pH had decreased to 2.56. The rapid voltage increase in these time intervals is the consequence of the progressively lower conductivities of the feed solution. This voltage increase results in a rapid increase in power consumption to convert residual sodium lactate. These results indicate that an economical process can be obtained by carrying out the electrodialysis to a partial conversion of at the most 98%.

However, under different conditions, in particular when the initial concentration of the monovalent lactate salt in the aqueous medium is of at least 30 wt.% or higher and of at most 40 wt.%, an electrodialysis up to 99 wt.% may be performed whilst still having an overall process which may be sufficiently economical. This may be the case, for instance, in methods where no vacuum distillation is performed to isolate lactic acid and other methods are chosen to recover lactic acid, e.g. the use of ion exchange resins. This may also be the case when higher starting concentrations are used and part of the water removal is performed before electrodialysis. In such situations the energy saved by not performing vacuum distillation and/or by performing water evaporation prior to water-splitting evaporation, may compensate the energy required to go up to a conversion higher than 98 wt.%, e.g. up to 99 wt.%, and still provide an economically viable process.

In the time interval of 180-210 minutes the current efficiency, calculated with the theoretical mass flows based on the current and Faradays law, and the actual mass flows based on analytical data of lactate, lactic acid and sodium was 0.68. In the time interval of 210-225 the current efficiency dropped to 0.64, and at conversions higher than 98% the current efficiency dropped to 0.36 or below. This means that at higher conversions the energy input itself increases but it also means that increasingly less of the electrical energy consumed is used for conversion.

### Example 2. Distillation of lactic acid from a solution of lactic acid and sodium lactate.

A solution of lactic acid and sodium lactate was prepared by adding 98.4 grams of a 60 wt.% food grade sodium lactate solution to 4310 grams of a 49 wt.% food grade lactic acid solution. This solution is representative of a mixture of lactic acid and sodium lactate that is obtained after electrodialysis with partial conversion and that is concentrated by evaporation of water. A glass lab Short Path Distillation (SPD) unit was used to concentrate this solution further, and subsequently distil lactic acid from it.

The lab SPD unit used was a glass KDL4 type unit, made by UIC. The SPD unit is essentially a double walled column, which can be heated with an oil bath to temperatures to above 100 °C. The liquid (i.e. the lactate/lactic acid feed solution) may be fed to the top of the SPD unit by pumping it with a peristaltic pump. The SPD unit is equipped with a top stirrer and wipers in such a way that a liquid film may be produced on the inner wall of the SPD unit column from the liquid that is pumped in from the top. The top of the SPD unit is also connected to a vacuum system, comprising a glass column, which is indirectly cooled through a double wall by a cold finger at -60 °C, an oil operated vacuum pump and a pressure control unit with a vacuum meter and a pressure valve. By applying vacuum and high temperature a relatively high boiling compound like lactic acid can be evaporated from the feed. The evaporated lactic acid can then be condensed in the SPD unit, which is equipped with an inner condenser cooled by water at 55 °C. The lactic acid can be collected in a glass bulb placed directly under the inner condenser of the SPD unit. The part of the feed that is not evaporated can be collected using an outlet on the side wall at the bottom of the double walled SPD column. Vapours that are not trapped on the inner condenser of the SPD unit are trapped in the glass column in the vacuum section operated with the cold finger.

First the feed solution was led over the SPD unit for dewatering. The oil temperature was 120 °C, the vacuum pressure 100 mbar, and the feed rate 10 ml/min. The inner condenser of the SPD unit was operated with tap water. A fraction of 336 g of a dewatered mixture of lactic acid and sodium lactate was collected, and immediately passed over the SPD unit again. Now the oil temperature was 130 °C, the vacuum pressure 6 mbar, the feed flow rate was 15 ml/min, and the inner condenser of the SPD unit was cooled to 55 °C. In this second SPD step at least half of the feed was distilled and recovered as a purified lactic acid, the overall recovery yield of lactic acid being of 65 % (based on the total weight of lactic acid present in the original feed). The feed to the SPD unit in this second step contained 5500-6000 ppm sodium, while the lactic acid product contained only 112 ppm sodium, i.e. a purification factor of about 49-54. Thus, the bulk of the lactic acid can be recovered in a pure form from the original mixture of lactic acid and sodium lactate using a short path distillation (SPD) unit.

### Example 3 Partial electrodialysis of potassium lactate solution.

A pilot electrodialysis unit with multiple cell pairs of bipolar and cationic membranes was used in a batch experiment to prepare lactic acid from a model potassium lactate solution. The potassium lactate solution was obtained by diluting a food grade 60 wt.% potassium lactate solution (PURASAL^{®} HiPure P from Purac) to a 34 wt.% concentration of potassium lactate by addition of water.

In a batch mode, a 20 kg feed of the 34 wt.% potassium lactate solution was converted to lactic acid via water-splitting electrodialysis in said pilot electrodialysis unit. The experiment lasted 76 min, after which a conversion of 88 mole% was obtained. The resulting solution had a lactic acid concentration of 26 wt.% and a residual potassium lactate concentration of 2.0 wt.%. In the first 70 minutes of the experiment the average voltage was about 42 V, and over the last 6 minutes the voltage rose up to 49 V, showing increased energy use at higher conversion. The average current efficiency was 0.78.

This experiment illustrates the suitability of a 34 wt.% monovalent lactate solution as a starting solution for obtaining lactic acid via water-splitting electrodialysis. Further, starting at 34 wt.% monovalent lactate concentration results in a lactic acid solution of 27 wt.%, which is more concentrated than solutions obtained when starting at lower concentrations of monovalent lactate salt (e.g. a solution of 17 wt.% lactic acid may be typically obtained when starting with a monovalent lactate salt solution of 21-23 wt.%). This means that in a process wherein the concentration of the starting monovalent lactate salt solution is relatively low (e.g. below 30 wt.%, below 25 wt.% or even below 10 wt.%) water may be preferably removed prior to electrodialysis to start with a higher monovalent lactate concentration, e.g. of at least 30 wt.% or higher (such as a 34 wt.% potassium lactate solution as in this example). As a result less water needs to be removed after performing water splitting electrodialysis. This may be the case when, e.g., the monovalent lactate salt is derived from a fermentation process.

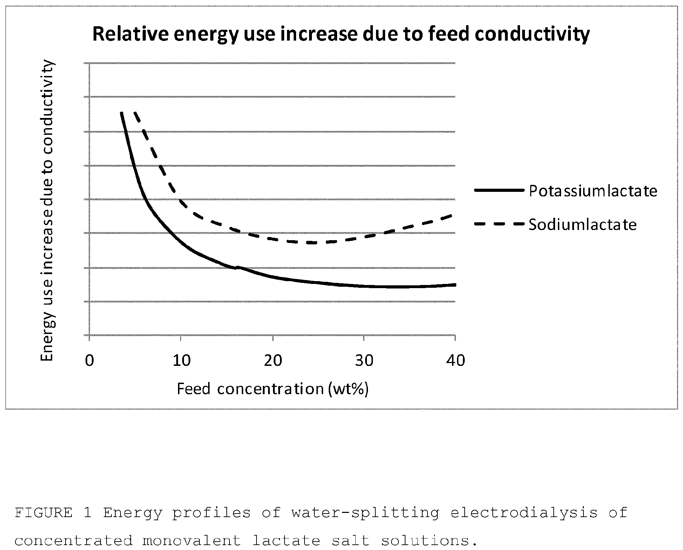

From the experiments performed and from the theoretical conductivities of monovalent lactate solutions (data and theoretical models not shown), it has been found that if a water-splitting electrodialysis of potassium lactate solutions is compared to a water-splitting electrodialysis of sodium lactate solutions under similar conditions, the increase of the energy use due to the conductivity of the solution is greater when starting with a sodium lactate solution (Figure 1). This means that under same conditions lower energy consumption may be achieved when working with potassium lactate solutions when compared to working with sodium lactate solutions. Further, as it can be derived from figure 1, an optimal energy low for potassium lactate is found when working at concentrations from 30 to 40 wt.% (Figure 1), in particular higher than 30wt.% and of at most 40 wt.%.

## Claims

1. Process for the preparation of lactic acid comprising the steps of:
a) providing an aqueous medium comprising magnesium lactate;
b) adding to the aqueous medium comprising magnesium lactate a monovalent base to form an aqueous medium comprising a water soluble monovalent lactate salt and a solid magnesium base;
c) separating the solid magnesium base from the aqueous medium comprising the water soluble monovalent lactate salt;
d) providing an aqueous medium comprising the water soluble monovalent lactate salt at a concentration of more than 30 wt.% and at most 45 wt.%;
e) subjecting the aqueous medium comprising the water soluble monovalent lactate salt from step d) to water-splitting electrodialysis, to produce a first solution comprising monovalent base and a second solution comprising lactic acid and monovalent lactate salt, the electrodialysis being carried out to a partial conversion of 40 to 99 mole%;
f) recovering lactic acid from the second solution comprising lactic acid and monovalent lactate salt.

2. Process according to claim 1, wherein in step d) an aqueous medium is provided comprising the water soluble monovalent lactate salt at a concentration of at least 32 wt.% and/or at most 40 wt.%, and/or from 32 to 38 wt%, even more preferably from 34 to 36 wt.%, and most preferred concentration an initial concentration of about 35 wt.%.

3. Process according to any one of the preceding claims, wherein step d) is a concentration step, a dilution step, or a step in which the concentration is not adjusted further, preferably a concentration step in which water is removed.

4. Process according to any one of the preceding claims, wherein recovering lactic acid from step f) comprises separating the second solution comprising lactic acid and monovalent lactate salt into lactic acid and a solution comprising monovalent lactate salt, wherein the separation is carried out through one or more of vapour-liquid separation, liquid-liquid separation, and solid-liquid separation.

5. Process according to claim 4, wherein the solution comprising monovalent lactate salt is recycled at least in part to earlier steps in the process so that it is provided to water-splitting electrodialysis, e.g., to one or more of steps b), c), d) and e).

6. Process according to claim 3 or 4, wherein the separation is carried out through vapour-liquid separation wherein the vapour-liquid separation comprises distillation, in particular a distillation carried out in a vacuum distillation unit.

7. Process according to any one of the preceding claims, wherein the water-splitting electrodialysis is carried out to a partial conversion of at least 50 mole%, in particular at least 60 mole%, more in particular at least 70 mole%, still more in particular at least 80 mole%, especially at least 85 mole%, or at least 90 mole% and/or at most 98 mole%, in particular 90-95 mole, or higher than 98 mole% and at most 99 mole%.

8. Process according to any one of the preceding claims, wherein the monovalent lactate salt is potassium lactate or sodium lactate, in particular potassium lactate.

9. Process according to any one of the preceding claims wherein the monovalent lactate salt is potassium lactate and in step d) an aqueous medium is provided comprising potassium lactate at a concentration of at least 32 wt.% and/or at most 40 wt.%, and/or from 32 to 38 wt.%, even more preferably from 34 to 36 wt.%, and most preferred concentration an initial concentration of about 35 wt.%.

10. Process according to any one of the preceding claims, wherein the water-splitting electrodialysis is carried out in an electrodialysis apparatus provided with a cation exchange membrane and a bipolar membrane.

11. Process according to any one of the preceding claims, wherein the aqueous medium comprising the magnesium lactate is provided by fermentation, wherein a carbohydrate source is fermented by means of a micro-organism to form lactic acid, a magnesium base being added as neutralising agent during fermentation to provide the magnesium lactate.

12. Process according to claim 11, wherein the magnesium base is magnesium hydroxide.

13. Process according to claim 11 or 12, wherein the aqueous medium comprising the magnesium lactate is subjected to a separation step to remove microbial cell matter prior to step b).

14. Process according to any one of the preceding claims, wherein the lactic acid obtained after the recovery step f) comprises at least 95 wt.% of lactic acid, in particular at least 97 wt.% of lactic acid, more in particular at least 99 wt.% of lactic acid, at least 99.5 wt.%, and most in particular at least 99.9 wt.% of lactic acid.

15. Process for the preparation of lactide and/or polylactic acid, comprising preparing lactic acid using the process according to any one of claims 1 to 14 and reacting the lactic acid to form lactide and/or polylactic acid.

## Patentansprüche

1. Verfahren zum Herstellen von Milchsäure, das die Schritte aufweist:
a) Bereitstellen eines wässrigen Mediums, das Magnesiumlaktat aufweist;
b) Zugeben einer monovalenten Base zu dem das Magnesiumlaktat aufweisenden wässrigen Medium, um ein wässriges Medium zu bilden, das ein wasserlösliches monovalentes Laktatsalz und einer festen Magnesiumbase aufweist;
c) Abtrennen der festen Magnesiumbase von dem das wasserlösliche monovalente Laktatsalz aufweisenden wässrigen Medium;
d) Bereitstellen eines wässrigen Mediums, das das wasserlösliche monovalente Laktatsalz in einer Konzentration von mehr als 30 Gew.-% und höchstens 45 Gew.-% aufweist;
e) Unterziehen des das wasserlösliche monovalente Laktatsalz aus Schritt d) aufweisenden wässrigen Mediums einer wasserspaltenden Elektrodialyse, um eine erste Lösung, die eine monovalente Base aufweist, und eine zweite Lösung zu bilden, die Milchsäure und monovalentes Laktatsalz aufweist, wobei die Elektrodialyse bis zu einer teilweisen Konversion von 40 bis 99 Mol-% durchgeführt wird;
f) Gewinnen von Milchsäure aus der Milchsäure und monovalentes Laktatsalz aufweisenden zweiten Lösung.

2. Verfahren nach Anspruch 1, wobei in Schritt d) ein wässriges Medium bereitgestellt wird, das das wasserlösliche monovalente Laktatsalz in einer Konzentration von zumindest 32 Gew.-% und/oder höchstens 40 Gew.-%, und/oder von 32 bis 38 Gew.-%, noch bevorzugter von 34 bis 36 Gew.-%, und als bevorzugteste Konzentration eine anfängliche Konzentration von etwa 35 Gew.-% aufweist.

3. Verfahren nach irgendeinem der vorhergehenden Ansprüche, wobei Schritt d) ein Konzentrationsschritt, ein Verdünnungsschritt oder ein Schritt ist, bei dem die Konzentration nicht weiter angepasst wird, vorzugsweise ein Konzentrationsschritt, bei dem Wasser entfernt wird.

4. Verfahren nach irgendeinem der vorhergehenden Ansprüche, wobei die Gewinnung von Milchsäure aus Schritt f) ein Trennen der zweiten Lösung, die Milchsäure und monovalentes Laktatsalz aufweist, in Milchsäure und eine Lösung, die monovalentes Laktatsalz aufweist, wobei die Trennung durch eine oder mehrere der folgenden Verfahren durchgeführt wird: Dampf-Flüssig-Trennung, Flüssig-Flüssig-Trennung und Fest-Flüssig-Trennung.

5. Verfahren nach Anspruch 4, wobei die Lösung, die monovalentes Laktatsalz aufweist, zumindest teilweise zu früheren Schritten in dem Verfahren zurückgeführt wird, so dass sie der wasserspaltenden Elektrodialyse bereitgestellt wird, beispielsweise zu einem oder mehreren der Schritte b), c), d) und e).

6. Verfahren nach Anspruch 3 oder 4, wobei die Trennung durch Dampf-Flüssig-Trennung durchgeführt wird, wobei die Dampf-Flüssig-Trennung eine Destillation aufweist, insbesondere eine Destillation, die in einer Vakuumdestillationseinheit durchgeführt wird.

7. Verfahren nach irgendeinem der vorhergehenden Ansprüche, wobei die wasserspaltende Elektrodialyse bis zu einer teilweisen Konversion von zumindest 50 Mol-%, insbesondere zumindest 60 Mol-%, weiter insbesondere zumindest 70 Mol-%, noch weiter insbesondere zumindest 80 Mol-%, besonders zumindest 85 Mol-%, oder zumindest 90 Mol-% und/oder höchstens 98 Mol-%, insbesondere 90-95 Mol, oder höher als 98 Mol-% und höchstens 99 Mol-%, durchgeführt wird.

8. Verfahren nach irgendeinem der vorhergehenden Ansprüche, wobei das monovalente Laktatsalz Kaliumlaktat oder Natriumlaktat, insbesondere Kaliumlaktat, ist.

9. Verfahren nach irgendeinem der vorhergehenden Ansprüche, wobei das monovalente Laktatsalz Kaliumlaktat ist und in Schritt d) ein wässriges Medium bereitgestellt wird, das Kaliumlaktat in einer Konzentration von zumindest 32 Gew.-% und/oder höchstens 40 Gew.-%, und/oder von 32 bis 38 Gew.-%, noch bevorzugter von 34 bis 36 Gew.-%, und als bevorzugteste Konzentration eine anfängliche Konzentration von etwa 35 Gew.-% aufweist.

10. Verfahren nach irgendeinem der vorhergehenden Ansprüche, wobei die wasserspaltende Elektrodialyse in einer Elektrodialysevorrichtung durchgeführt wird, die mit einer Kationenaustauschmembran und einer bipolaren Membran bereitgestellt ist.

11. Verfahren nach irgendeinem der vorhergehenden Ansprüche, wobei das wässrige Medium, das Magnesiumlaktat aufweist, durch Fermentation bereitgestellt wird, wobei eine Kohlenhydratquelle mittels eines Mikroorganismus fermentiert wird, um Milchsäure zu bilden, wobei eine Magnesiumbase als Neutralisationsmittel während der Fermentation zugefügt wird, um das Magnesiumlaktat bereitzustellen.

12. Verfahren nach Anspruch 11, wobei die Magnesiumbase Magnesiumhydroxid ist.

13. Verfahren nach Anspruch 11 oder 12, wobei das wässrige Medium, das das Magnesiumlaktat aufweist, einem Trennungsschritt unterzogen wird, um mikrobielles Zellmaterial vor Schritt b) zu entfernen.

14. Verfahren nach irgendeinem der vorhergehenden Ansprüche, wobei die Milchsäure, die nach dem Gewinnungsschritt f) erhalten wird, zumindest 95 Gew.-% Milchsäure, insbesondere zumindest 97 Gew.-% Milchsäure, weiter insbesondere zumindest 99 Gew.-% Milchsäure, zumindest 99,5 Gew.-%, und noch weiter insbesondere zumindest 99,9 Gew.-% Milchsäure aufweist.

15. Verfahren zur Herstellung von Laktid und/oder Polymilchsäure, mit Herstellen von Milchsäure unter Verwendung des Verfahrens nach irgendeinem der Ansprüche 1 bis 14 und Umsetzen der Milchsäure, um Laktid und/oder Polymilchsäure zu bilden.

## Revendications

1. Procédé de préparation d'acide lactique comprenant les étapes consistant à :
a) fournir un milieu aqueux comprenant du lactate de magnésium ;
b) ajouter au milieu aqueux comprenant du lactate de magnésium une base monovalente pour former un milieu aqueux comprenant du sel de lactate monovalent soluble dans l'eau et une base de magnésium solide ;
c) séparer la base de magnésium solide du milieu aqueux comprenant le sel de lactate monovalent soluble dans l'eau ;
d) fournir un milieu aqueux comprenant le sel de lactate monovalent soluble dans l'eau en une concentration de plus de 30 % en poids et de 45 % en poids au maximum ;
e) soumettre le milieu aqueux comprenant le sel de lactate monovalent soluble dans l'eau provenant de l'étape d) à une électrodialyse par dissociation d'eau, pour produire une première solution comprenant une base monovalente et une seconde solution comprenant de l'acide lactique et du sel de lactate monovalent, l'électrodialyse étant réalisée à une conversion partielle de 40 à 99 % en mole ;
f) récupérer de l'acide lactique à partir de la seconde solution comprenant de l'acide lactique et du sel de lactate monovalent.

2. Procédé selon la revendication 1, dans lequel, à l'étape d), un milieu aqueux est fourni comprenant le sel de lactate monovalent soluble dans l'eau en une concentration d'au moins 32 % en poids et/ou de 40 % en poids au maximum, et/ou de 32 à 38 % en poids, encore plus préférablement de 34 à 36 % en poids, et la concentration préférée entre toutes une concentration initiale d'environ 35 % en poids.

3. Procédé selon l'une quelconque des revendications précédentes, dans lequel l'étape d) est une étape de concentration, une étape de dilution ou une étape dans laquelle la concentration n'est pas ajustée davantage, de préférence une étape de concentration dans laquelle l'eau est retirée.

4. Procédé selon l'une quelconque des revendications précédentes, dans lequel la récupération d'acide lactique provenant de l'étape f) comprend la séparation de la seconde solution comprenant de l'acide lactique et du sel de lactate monovalent en acide lactique et une solution comprenant du sel de lactate monovalent, dans lequel la séparation est réalisée par le biais d'une ou plusieurs parmi une séparation vapeur-liquide, une séparation liquide-liquide et une séparation solide-liquide.

5. Procédé selon la revendication 4, dans lequel la solution comprenant du sel de lactate monovalent est recyclée au moins en partie à des étapes antérieures du procédé de sorte qu'elle soit fournie à l'électrodialyse par dissociation d'eau, par exemple à une ou plusieurs des étapes b), c), d) et e).

6. Procédé selon la revendication 3 ou 4, dans lequel la séparation est réalisée par le biais d'une séparation vapeur-liquide, dans lequel la séparation vapeur-liquide comprend une distillation, particulièrement une distillation réalisée dans une unité de distillation sous vide.

7. Procédé selon l'une quelconque des revendications précédentes, dans lequel l'électrodialyse par dissociation d'eau est réalisée à une conversion partielle d'au moins 50 % en mole, particulièrement d'au moins 60 % en mole, plus particulièrement d'au moins 70 % en mole, encore plus particulièrement d'au moins 80 % en mole, spécialement d'au moins 85 % en mole, ou d'au moins 90 % en mole et/ou de 98 % en mole au maximum, particulièrement de 90-95 moles, ou de plus de 98 % en mole et de 99 % en mole au maximum.

8. Procédé selon l'une quelconque des revendications précédentes, dans lequel le sel de lactate monovalent est du lactate de potassium ou du lactate de sodium, particulièrement du lactate de potassium.

9. Procédé selon l'une quelconque des revendications précédentes dans lequel le sel de lactate monovalent est du lactate de potassium et à l'étape d) un milieu aqueux est fourni comprenant du lactate de potassium en une concentration d'au moins 32 % en poids et/ou de 40 % en poids au maximum, et/ou de 32 à 38 % en poids, encore plus préférablement de 34 à 36 % en poids, et la concentration préférée entre toutes une concentration initiale d'environ 35 % en poids.

10. Procédé selon l'une quelconque des revendications précédentes, dans lequel l'électrodialyse par dissociation d'eau est réalisée dans un appareil d'électrodialyse doté d'une membrane échangeuse de cations et d'une membrane bipolaire.

11. Procédé selon l'une quelconque des revendications précédentes, dans lequel le milieu aqueux comprenant le lactate de magnésium est fourni par fermentation, dans lequel une source de carbohydrates est fermentée au moyen d'un micro-organisme pour former de l'acide lactique, une base de magnésium étant ajoutée en tant qu'agent neutralisant pendant la fermentation pour fournir le lactate de magnésium.

12. Procédé selon la revendication 11, dans lequel la base de magnésium est de l'hydroxyde de magnésium.

13. Procédé selon la revendication 11 ou 12, dans lequel le milieu aqueux comprenant le lactate de magnésium est soumis à une étape de séparation pour éliminer une matière cellulaire microbienne avant l'étape b).

14. Procédé selon l'une quelconque des revendications précédentes, dans lequel l'acide lactique obtenu après l'étape de récupération f) comprend au moins 95 % en poids d'acide lactique, particulièrement au moins 97 % en poids d'acide lactique, plus particulièrement au moins 99 % en poids d'acide lactique, au moins 99,5 % en poids, et le plus particulièrement au moins 99,9 % en poids d'acide lactique.

15. Procédé de préparation de lactide et/ou d'acide polylactique, comprenant la préparation d'acide lactique en utilisant le procédé selon l'une quelconque des revendications 1 à 14 et la réaction de l'acide lactique pour former du lactide et/ou de l'acide polylactique.
